# EUROPÄISCHE PATENTSCHRIFT

(11) **EP 1 386 081 B1**
(45) Veröffentlichungstag und Bekanntmachung des Hinweises auf die Patenterteilung: **23.08.2006**
(21) Anmeldenummer: 02724301.3
(22) Anmeldetag: 29.04.2002
(51) Int. Cl.: F04D 29/04, H02K 7/09

(54) **VERFAHREN ZUR LAGEREGELUNG EINES PERMANENTMAGNETISCH GELAGERTEN ROTIERENDEN BAUTEILS**
METHOD FOR ADJUSTING THE POSITION OF A ROTATING COMPONENT WHICH IS BORNE BY MEANS OF A PERMANENT-MAGNET
PROCEDE POUR REGULER LA POSITION D'UNE PIECE ROTATIVE, MONTEE AU MOYEN D'AIMANTS PERMANENTS

(30) Priorität: 30.04.2001 DE 10123138
(43) Veröffentlichungstag der Anmeldung: 04.02.2004
(73) Patentinhaber: Berlin Heart AG, 12247 Berlin (DE)
(72) Erfinder: HOFFMANN, Jan, 12207 Berlin (DE); ARNDT, Andreas, 12489 Berlin (DE); MERKEL, Tobias, 14532 Kleinmachnow (DE)
(74) Vertreter: Golkowsky, Stefan
(86) Internationale Anmeldenummer: PCT/EP2002/004737
(87) Internationale Veröffentlichungsnummer: WO 2002/088548

(56) Entgegenhaltungen:
- WO-A-00/64030
- WO-A-00/74748
- US-A- 5 385 581
- US-A- 6 015 272

## Beschreibung

Die Erfindung betrifft ein Verfahren zur Lageregelung eines permanentmagnetisch gelagerten rotierenden Bauteils, z. B. des Rotors eines bürstenlosen Synchronmotors, mit Hilfe einer Positionsbestimmung dieses Bauteils durch eine Lagesensorik und zusätzlicher, das Magnetfeld der permanentmagnetischen Lagerung beeinflussender Steuerstromspulen, deren Stromhöhe durch die Position des Bauteils bestimmt wird. Der Synchronmotor kann beispielsweise als Antrieb für eine axiale Fluidförderpumpe dienen.

Mehrphasige Fluide, z.B. Emulsionen und Dispersionen mit geringer Stabilität, können bei Förderung in entsprechenden Fördersystemen leicht in instabile Bereiche gelangen.

Ein besonders empfindliches Fluid stellt Blut dar. Blut ist im natürlichen Kreislaufsystem hermetisch von der Umwelt abgeschirmt, so dass es keinen Fremdeinflüssen unterliegt. Besteht jedoch die Notwendigkeit, das Herz durch eine künstliche Blutpumpe zu ersetzen oder den Kreislauf durch eine zusätzliche Herzpumpe zu unterstützen, so kommt es zu Wechselwirkungen des Blutes mit dem technischen System. Das Blut unterliegt dann leicht der Hämolyse oder Thrombenbildung mit den entsprechenden nachteiligen Wirkungen für den Patienten. Es sind deshalb in jüngster Zeit verstärkt Anstrengungen unternommen worden, um Fluidförderpumpen so auszubilden, dass es zu möglichst wenig mechanischen Einflüssen auf das Blut bzw. auf andere empfindliche Fluide kommt. Eine Möglichkeit hierzu ist die magnetische Lagerung des rotierenden Elements eines Pumpenantriebes. Der Vorteil der magnetischen Lagerung besteht nicht nur darin, dass keine mechanisch miteinander reibenden Teile mehr vorhanden sind, sondern dass auch die erreichbare Drehbeschleunigung des rotierenden Elements erhöht und die Regelbarkeit der Drehzahl und damit des Volumenstromes verbessert werden können.

Eine solche Fluidpumpe lässt sich üblicherweise in einen bürstenlosen Synchronmotor integrieren. Sie besteht nach der WO 00/640 30 im wesentlichen aus einem zylindrischen Rohr, das beidseitig mit dem Fluidsystem verbunden werden kann. Das Rohr wird von dem Stator, bestehend aus Blechpaket, Wicklung und Eisenrückschlußkappen umgeben. Der Rotor enthält permanentmagnetische Felderreger und weist an seinem Außenmantel Fördereinrichtungen für das Fluid auf, so dass dieses im Ringraum zwischen dem Rohr und dem Rotor axial gefördert wird.

Der Rotor wird magnetisch gelagert. Er trägt zu diesem Zweck an seinen beiden Stirnseiten angebrachte zylinderförmige oder ringförmige Permanentmagnete, die in axialer Richtung magnetisiert sind. Den Permanentmagneten des Rotors stehen gegensinnig magnetisierte Permanentmagnete gegenüber, die z.B. in den Stirnseiten von Leiteinrichtungen angeordnet sein können, die ihrerseits in dem zylindrischen Rohr befestigt sind.

In radialer Richtung wirken beide Magnetpaare, wenn sie auf gegenseitige Anziehung orientiert sind, stabilisierend, das heißt die radiale Lagerung ist passiv stabil. In axialer Richtung ist der Rotor jedoch instabil.

Ohne zusätzliche Stabilisierung würde der Rotor von einem der beiden Permanentmagnetpaare angezogen werden. Es werden deshalb Steuerspulen an den Statorseiten so angeordnet, dass ein Strom durch die in Reihe geschalteten Steuerspulen das Magnetfeld des einen Permanentmagnetpaares abschwächt und das des anderen verstärkt. Der Steuerstrom muss in Abhängigkeit von der aktuellen axialen Rotorposition geregelt werden. Hierzu muss die Rotorposition mit Hilfe von Lagesensoren ermittelt werden.

Die Lagesensoren bestehen beispielsweise aus zwei Sensorspulen, die an den Stirnseiten der Leiteinrichtungen angeordnet sein können. Diesen gegenüber liegen an den Stirnseiten des Rotors Aluminiumkörper, in denen sich Wirbelströme bilden, wenn die Sensorspulen mit einem Wechselstrom beaufschlagt werden. Bei der axialen Bewegung des Rotors kommt es dann zu einer Änderung der Induktivität der Sensorspulen, die sich bei Anordnung in einer Brückenschaltung zu einem Messsignal für die Rotorposition auswerten lässt.

Da insbesondere bei einem pulsatilen Durchfluss durch die Pumpe laufend Störkräfte auf den Rotor ausgeübt werden, muss die Lageregelung eine veränderte axiale Rotorposition sehr schnell ausregeln können. Andererseits soll der Steuerstrom eine geringe Verlustleistung verursachen, was insbesondere für Blutpumpen von Bedeutung ist, da die erzeugte Wärmeenergie möglichst klein gehalten werden soll. Außerdem muss die Antriebsenergie implantierten Batterien entnommen werden, deren Betrieb möglichst lange anhalten soll.

Der Erfindung liegt die Aufgabe zu Grunde, ein Verfahren zur Lageregelung eines magnetisch gelagerten Bauteils anzugeben, mit dem die Verlustleistung der Lageregelung klein gehalten werden kann.

Erfindungsgemäß wird die Aufgabe durch die Merkmale des Anspruches 1 gelöst. Zweckmäßige Ausgestaltungen sind Gegenstand der Unteransprüche.

Danach wird der Strom durch die Steuerstromspulen pulsweitenmoduliert durch einen von einem der Lagesensorik nachgeordneten Regler vorgegebenen Sollwert geregelt, wobei bei hohem Sollwert auf eine höhere Spannungsstufe umgeschaltet wird. Das hat den Vorteil, dass die Regelzeiten sehr klein gehalten werden können und die benötigte Leistung trotzdem gering bleibt.

Der Ist-Wert der Lagesensorik wird für eine definierte Dauer, jeweils beginnend spätestens mit der Schaltflanke des Steuerstromes, gespeichert und die Lagesensorik für diesen Zeitraum außer Betrieb gesetzt.

In vorteilhafter Weise wird bei Verwendung der Lageregelung in einem bürstenlosen Synchronmotor der Ist-Wert der Lagesensorik auch hinsichtlich der Ansteuerimpulse der Motorspulen für eine definierte Dauer, beginnend spätestens mit der Schaltflanke der Ansteuerimpulse zwischengespeichert und die Lagesensorik für diesen Zeitraum außer Betrieb gesetzt.

Die durch die Taktung entstehenden Störungen hinsichtlich der Positionsbestimmung werden durch Ausblenden der Messung während dieser Zeiten und die Speicherung der Messwerte beherrscht.

Für bestimmte Anwendungen kann es zweckmäßig sein, dass aus dem Sollwert des der Lagesensorik nachgeordneten Reglers das Quadrat gebildet und bei einer zeitgemittelten Schwellwertüberschreitung dieses Wertes die Lageregelung bis zur nächsten Schwellwertunterschreitung außer Betrieb gesetzt wird. Dadurch wird die Erwärmung der Steuerspulen nachgebildet und so eine Überhitzung vermieden.

Zweckmäßig wird für den der Lagesensorik nachgeordneten Regler ein PID-Regler mit I₂-Anteil verwendet.

Die Erfindung soll nachstehend anhand eines Ausführungsbeispiels näher erläutert werden. In den zugehörigen Zeichnungen zeigen:
- Fig. 1: einen Querschnitt durch eine Fluidförderpumpe, die für die Durchführung des erfindungsgemäßen Verfahrens geeignet ist,
- Fig. 2: eine Prinzipdarstellung der Lageregelung mit der efindungsgemäßen zusätzlichen Stromregelung und
- Fig. 3: ein Blockschaltbild des Lagereglers.

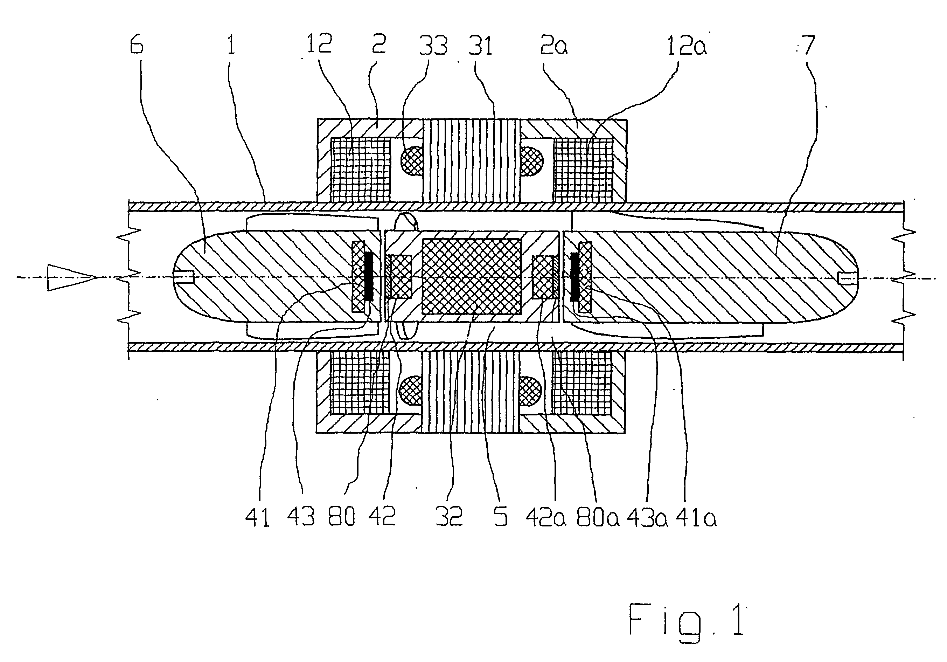

Fig. 1 zeigt eine solche zur Durchführung des Verfahrens geeignete axiale Pumpe. Der Antrieb der Blutpumpe arbeitet nach dem Prinzip eines elektronisch kommutierten Synchronmotors. Der Motor hat einen Stator, bestehend aus einem Blechpaket 31, Wicklungen 33 und Eisenrückschlußkappen 2, 2a, und einem Rotor 5 mit permanentmagnetischem Kern 32. Der Stator umschließt einen rohrförmigen Hohlkörper 1, in dem in axialer Richtung ein Fluid, im vorliegenden Falle also Blut, gefördert wird. Der Rotor 5 ist berührungsfrei magnetisch gelagert.

Das magnetische Lager besteht aus Permanentmagneten 42, 42a an den Rotorstirnseiten und Permanentmagneten 41, 41a an den Stirnseiten von Leiteinrichtungen 6 und 7. Die Leiteinrichtungen 6, 7 sind an der Innenwand des rohrförmigen Hohlkörpers 1 befestigt.

Zum magnetischen Lager gehören außerdem Steuerspulen 12, 12a. Zur Messung der aktuellen Rotorposition dienen Sensorspulen 43, 43a in den Leiteinrichtungen 6, 7 und diesen gegenüberliegende Kurzschlußringe 80, 80a.

Die Paare der Permanentmagneten 41, 42; 41a, 42a sind jeweils auf Anziehung gepolt. Magnetisch liegen die Paare in Reihe.

Ohne eine zusätzliche Stabilisierung würde der Rotor 5 zu einer Seite hin angezogen werden, es besteht in axialer Richtung ein instabiles Gleichgewicht. In radialer Richtung wirken beide Magnetpaare zentrierend, die radiale Lage ist somit passiv stabil.

Die Steuerspulen 12, 12a sind elektrisch in Reihe geschaltet und magnetisch so angeordnet, dass ein Strom das Magnetfeld des einen Magnetpaares schwächt und das des anderen verstärkt. Der magnetische Rückschluss geschieht über die Eisenrückschlußkappen 2, 2a und das Blechpaket 31 des Stators.

Die axiale Position des Rotors 5 kann mit Hilfe der Sensorspulen 43, 43a ermittelt werden. Die Sensorspulen 43, 43a werden mit einer höherfrequenten Spannung beaufschlagt. Bei axialer Bewegung des Rotors 5 kommt es zu einer Änderung der Induktivitäten der Sensorspulen 43, 43a. Durch Anordnung der Sensorspulen 43, 43a in einer Brückenschaltung lässt sich ein Messsignal für die axiale Position des Rotors 5 gewinnen.

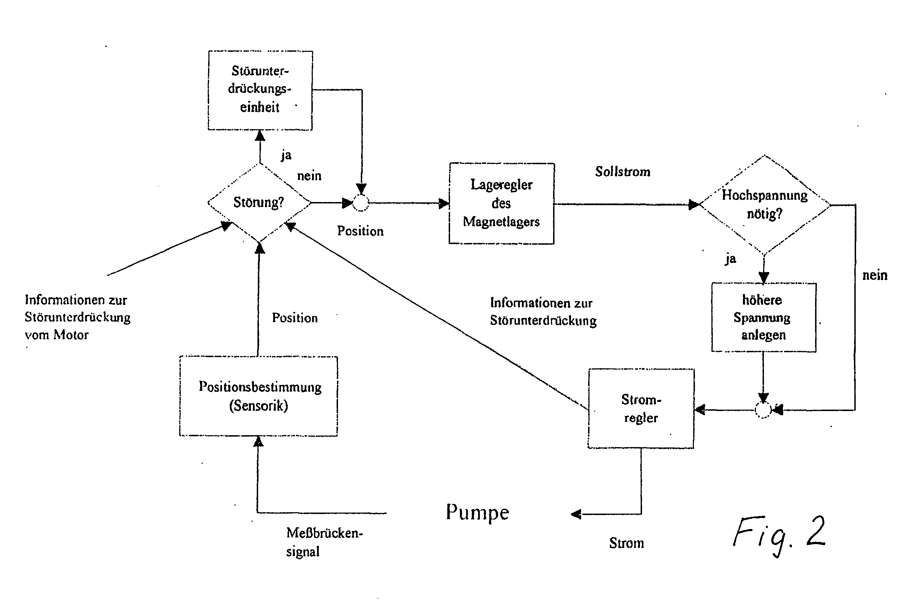

Wie Fig. 2 zeigt liegt am Ausgang eines der Lagesensorik nachgeordneten Reglers dann ein Stellwert für den Steuerstrom durch die Steuerspulen 12, 12a an. Der Steuerstrom wird über einen Stromregler in die Steuerspulen 12, 12a eingespeist. Der Stromregler arbeitet als geschlossener Regelkreis, indem er den Strom durch die Steuerspulen 12, 12a misst und mit der Vorgabe (Sollstrom) des Lagereglers vergleicht. Über eine Pulsweitenmodulation einer getakteten Leistungsstufe wird der tatsächliche Strom auf den Sollstrom abgeglichen. Dieser Vorgang benötigt eine bestimmte Zeit, die von der Differenz aus Sollstrom und tatsächlichem Strom abhängt. Je höher die Spannung ist, mit der die Leistungsstufe arbeitet, desto kürzer ist die Regelzeit des Stromreglers. Andererseits erhöht sich mit der Spannung auch die Verlustleistung in der Leistungsstufe. Damit ein schnelles Reagieren des Stromreglers und eine geringe Verlustleistung erreicht werden, wird eine höhere Spannung nur dann zugeschaltet, wenn tatsächlich eine große Differenz zwischen Soll- und Ist-Strom vorhanden ist, ansonsten wird mit niedriger Spannung gearbeitet.

Durch die Ansteuerung der Steuerspulen 12, 12a durch die getaktete Leistungsstufe entstehen in den Sensorspulen 43, 43a Störungen, die die Positionsbestimmung des Rotors 5 verfälschen können. Diese Störungen koppeln mit jeder Schaltflanke an den Steuerspulen 12, 12a auf die Sensorspulen 43, 43a über und klingen nach definierter Dauer ab. Deshalb wird für die erwartete Dauer dieser Störungen das unmittelbar zuvor gewonnene Positionssignal zwischengespeichert und die Positionsbestimmung ausgesetzt. Der Lagerregler arbeitet in dieser Zeit mit dem zwischengespeicherten Wert. Ist die Störung abgeklungen, wird wieder die Position mittels der Sensorspulen 43, 43a bestimmt. Ähnliche Störungen können auch durch die Ansteuerung der Motorspulen 33 entstehen. Auch dafür wird das Verfahren der Zwischenspeicherung angewendet. Die Elektronik zur Störunterdrückung erhält vom Stromregler und von der Ansteuerelektronik des Motors genau den Zeitpunkt des möglichen Einsetzens der Störungen mitgeteilt, damit sie die Speicherung des Positionssignals vornehmen kann.

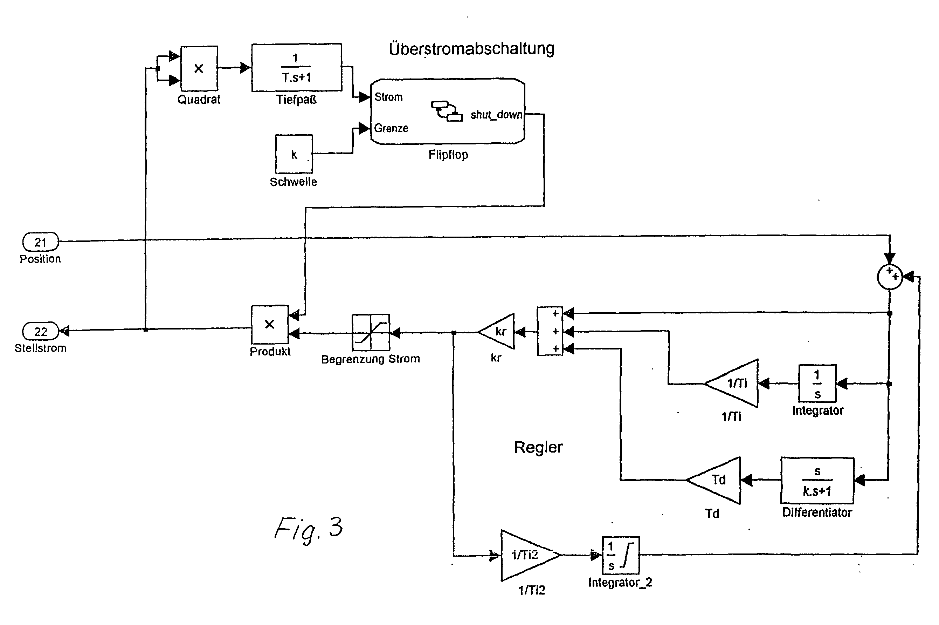

Fig. 3 zeigt die Schaltung für die Lageregelung des Magnetlagers. Aus der gemessenen Position des Rotors, die am Pfad 21 anliegt, wird ein Stellstrom für die Steuerspulen 12, 12a, der zu einem sicheren Schweben des Rotors 5 in allen Betriebszuständen führt, ermittelt und am Ausgang 22 des Lagereglers bereitgestellt. Der Lageregler besteht aus einem PID-Regler, der durch die Zeitkonstanten des Integrators Ti und des Differenziators Td sowie durch den Verstärkungsfaktor kr eines Regelverstärkers charakterisiert ist. Zum Schutz der Steuerspulen 12, 12a vor thermischer Überlastung wird zusätzlich aus dem Stromquadrat die zu erwartende Verlustleistung bestimmt. Bei einer über einen Tiefpass zeitgemittelten Schwellwertüberschreitung wird die Lageregelung ausgeschaltet, bis der Schwellwert wieder unterschritten wird. Der Lageregler hat als zusätzliche Vorgabe, den Strom durch die Steuerspulen 12, 12a so gering wie möglich zu halten. Über einen Integrator (I₂-Glied) wird der Stellstrom auf den Reglereingang zurückgekoppelt. Als Ergebnis positioniert sich der Rotor 5 immer an der axialen Stelle in der Pumpe, an der nur ein minimaler Strom durch die Steuerspulen 12, 12a fließt.

### Bezugszeichenliste

- 2: Eisenrückschlußkappe
- 2a: Eisenrückschlußkappe
- 5: Rotor
- 6: Leiteinrichtung
- 7: Leiteinrichtung
- 12: Steuerspule
- 12a: Steuerspule
- 31: Blechpaket
- 33: Wicklungen
- 41: Permanentmagnet
- 41a: Permanentmagnet
- 42: Permanentmagnet
- 42a: Permanentmagnet
- 43: Sensorspule
- 43a: Sensorspule
- 21: Pfad
- 22: Ausgang
- 80: Kurzschlussring
- 80°: Kurzschlussring
- Ti: Integrator
- Td: Differentiator
- kr: Verstärkungsfaktor

## Patentansprüche

1. Verfahren zur Lageregelung eines permanentmagnetisch gelagerten rotierenden Bauteils (5) mit Hilfe einer Positionsbestimmung dieses Bauteils durch eine Lagesensorik und zusätzlicher, das Magnetfeld der permanentmagnetischen Lagerung beeinflussender Steuerstromspulen, deren Stromhöhe durch die Position des Bauteils bestimmt wird,
**dadurch gekennzeichnet, dass**
der Strom durch die Steuerstromspulen pulsweitenmoduliert durch einen von einem der Lagesensorik nachgeordneten Regler vorgegebenen Sollwert geregelt wird, bei hohem Sollwert auf eine höhere Spannungsstufe umgeschaltet wird und der Ist-Wert der Lagesensorik für eine definierte Dauer, jeweils beginnend spätestens mit der Schaltflanke des Steuerstromes, gespeichert und die Lagesensorik für diesen Zeitraum außer Betrieb gesetzt wird.

2. Verfahren nach Anspruch 1,
**dadurch gekennzeichnet, dass**
der Ist-Wert der Lagesensorik bei Verwendung in einem bürstenlosen Synchronmotor auch hinsichtlich der Ansteuerimpulse der Motorspulen für eine definierte Dauer, beginnend spätestens mit der Schaltflanke der Ansteuerimpulse zwischengespeichert und die Lagesensorik für diesen Zeitraum außer Betrieb gesetzt wird.

3. Verfahren nach Anspruch 1 oder 2,
**dadurch gekennzeichnet, dass**
aus dem Sollwert des der Lagesensorik nachgeordneten Reglers das Quadrat gebildet und bei einer zeitgemittelten Schwellwertüberschreitung dieses Wertes die Lageregelung bis zur nächsten Schwellwertunterschreitung außer Betrieb gesetzt wird.

4. Verfahren nach einem der vorhergehenden Ansprüche,
**dadurch gekennzeichnet, dass**
für den der Lagesensorik nachgeordneten Regler ein PID-Regler mit I₂-Anteil verwendet wird.

## Claims

1. Method for adjusting the position of a rotating component (5) which is borne by means of a permanent magnet with the aid of position determination of said component by means of a position sensor and additional control coils, the current strength of which is determined by the position of the component, and which influences the magnetic field of the permanent magnet bearing, **characterised in that** the current through the control coils is pulse-width modulated by means of a target value generated by a controller connected to the output of the position sensor, and is switched to a higher voltage step if the target value is high, and **in that** the actual value of the position sensor is stored for a defined period beginning in each case, at the latest, with the switching edge of the control current and **in that** the position sensor is de-activated for said period.

2. Method according to Claim 1, **characterised in that** the actual value of the position sensor is stored for a defined period beginning, at the latest, with the switching edge of the control pulse, also with respect to the control impulses for the motor windings when used in a brushless synchronous motor, and **in that** the position sensor is de-activated for said period.

3. Method according to Claim 1 or 2, **characterised in that** the square of the target value of the controller connected to the output of the position sensor is calculated, and **in that** the position sensor is de-activated whenever an average value, calculated over time, exceeds this threshold value for this target value and is kept deactivated until the value drops to below the threshold value.

4. Method according to one of the preceding Claims, **characterised in that** a PID controller with I₂ content is used as the controller that receives the position sensor output.

## Revendications

1. Procédé de réglage de la position d'une pièce (5) rotative montée dans un palier magnétique permanent, au moyen d'une détermination de la position de cette pièce par un système de capteurs de position et de bobines de courant de commande supplémentaires, qui influencent le champ magnétique du palier magnétique permanent et dont l'intensité de courant est déterminée par la position de la pièce, **caractérisé en ce que** le courant à travers les bobines de courant de commande est réglé avec une modulation de l'intervalle des impulsions par une valeur de consigne prédéfinie par un régulateur monté en aval du système de capteurs de position, est commuté à un niveau de tension plus élevé en présence d'une valeur de consigne élevée, et la valeur réelle du système de capteurs de position est mémorisée pendant une période définie, commençant dans chaque cas au plus tard avec le flanc de l'impulsion de commutation du courant de commande, et pendant cet intervalle de temps le système de capteurs de position est mis hors service.

2. Procédé selon la revendication 1, **caractérisé en ce que** la valeur réelle du système de capteurs de position, en cas d'utilisation d'un moteur synchrone sans balais, est aussi mémorisée temporairement pour les impulsions d'activation des bobines du moteur pendant une durée définie, commençant dans chaque cas au plus tard avec le flanc des impulsions d'activation et, pendant cet intervalle de temps, le système de capteurs de position est mis hors service.

3. Procédé selon la revendication 1 ou 2, **caractérisé en ce que** la valeur de consigne du régulateur monté en aval du système de capteurs de position est élevée au carré et, lorsque cette valeur est supérieure à une valeur seuil moyenne en fonction du temps, le système de réglage de la position est mis hors service jusqu'à ce que cette valeur soit à nouveau inférieure à la valeur seuil.

4. Procédé selon l'une quelconque des revendications précédentes, **caractérisé en ce que**, pour le régulateur monté en aval du système de capteurs de position, on utilise un régulateur PID avec une fraction I₂.
